# EUROPÄISCHE PATENTSCHRIFT

(11) **EP 1 620 179 B1**
(45) Veröffentlichungstag und Bekanntmachung des Hinweises auf die Patenterteilung: **11.10.2006**
(21) Anmeldenummer: 04728733.9
(22) Anmeldetag: 22.04.2004
(51) Int. Cl.: A61N 2/12

(54) **ERFASSUNG BZW. BEEINFLUSSUNG PHYSIOLOGISCHER UND/ODER PATHOLOGISCHER ZUSTÄNDE**
DETECTION AND INFLUENCING OF PHYSIOLOGICAL AND/OR PATHOLOGICAL STATES
DETECTION D'ETATS PHYSIOLOGIQUES ET/OU PATHOLOGIQUES ET INTERVENTION SUR CES ETATS

(30) Priorität: 24.04.2003 AT 6252003
(43) Veröffentlichungstag der Anmeldung: 01.02.2006
(73) Patentinhaber: Korpan, Nikolai, 1190 Wien (AT); Filippov, Juri A., Mechnikova str. 7/8 Dnipropetrovsk, 320070 (UA)
(72) Erfinder: Korpan, Nikolai, 1190 Wien (AT); Filippov, Juri A., Mechnikova str. 7/8 Dnipropetrovsk, 320070 (UA)
(74) Vertreter: Müllner, Martin
(86) Internationale Anmeldenummer: PCT/AT2004/000134
(87) Internationale Veröffentlichungsnummer: WO 2004/093992

(56) Entgegenhaltungen:
- EP-A- 0 838 236
- EP-A- 1 287 852
- WO-A-99/39769
- FR-A- 2 210 420
- US-A- 6 001 055

## Beschreibung

Die Erfindung betrifft eine Vorrichtung zur Erfassung bzw. Beeinflussung des physiologischen und ggf. pathologischen Zustands des menschlichen oder tierischen Körpers mit einem Gehäuse, das eine erste Gehäusewand aufweist, die eine Außenfläche besitzt, die dazu bestimmt ist, an den zu behandelnden Körper angelegt zu werden, wobei in dem Gehäuse ein Rotor angeordnet ist, der um eine Achse drehbar angetrieben ist, die im Wesentlichen senkrecht zu der ersten Gehäusewand ist, und wobei auf dem Rotor erste Magnete angeordnet sind, deren Magnetfelder gleichsinnig parallel zur Drehachse orientiert sind.

Es sind verschiedenartige Vorrichtungen bekannt geworden, die den menschlichen Körper durch Anwendung von elektromagnetischen Feldern zu beeinflussen. Teilweise handelt es sich dabei um diagnostische Einrichtungen, wie etwa bei der Kernspintomographie, zum anderen Teil aber stehen therapeutische Anwendungen im Vordergrund.

Es ist eine Vorrichtung bekannt geworden, um den biologischen Zustand eines Patienten durch Messung von abgestrahlten Wellen im Millimeterbereich zu messen. Solche Verfahren sind beispielsweise veröffentlicht in: Devyatkov N.D., Golant M.V.: "About outlooks of usage of electromagnetic radiations of a millimetre wave in quality of high-informative means of obtaining of data about specific processes in alive organisms" in Letters to Journal of Technical Physics, 1986; 12(5):288-291.

Eine solche direkte Messung der Strahlungsintensität ist jedoch aufgrund der sehr kleinen Amplituden schwierig und mit hohem apparativen Aufwand verbunden. Der praktischen Anwendung solcher Verfahren sind daher enge Grenzen gesetzt.

Weitere bekannte Diagnostizierverfahren versuchen Erkenntnisse in nicht-invasiver Art zu gewinnen, indem die Frequenz und Periodizität von grundlegenden physiologischen Vorgängen gemessen werden. Dabei kann es sich um den Atemrhythmus oder um den Herzrhythmus bzw. Pulsschlag handeln. Im Zuge solcher Messungen kann der zu untersuchende Körper mit hochfrequenten Wellen belastet werden, um zusätzliche Erkenntnisse zu gewinnen. Auf diese Weise können bestimmte Diagnosen gestellt werden, es ist jedoch nicht möglich, beispielsweise die Gehirnaktivität in geeigneter Weise zu erfassen.

Weiters sind magnetoakustische Vorrichtungen für die nicht-invasive Messung von bioelektrischen Strömen im Gehirn bekannt geworden (Towe B.C., Islam M.R.: "A magneto-acoustic device for the non-invasive measurements of bioelectric currents"; IEEE Trans. Biomed. Eng. 1988; 35(10):892-894; Spiegel R.I. e.a. Measurements of small mechanical vibration of brain tissue exposed to extremely low-frequency electric fields; Bioelectromagnetic, 1986; 7(3):295-306). Solche nicht-invasiven magnetoakustischen Messungen können - auf der Basis der Durchführung unter Belastung des Organismus mit einem variablen elektromagnetischen Feld - eine große Informationsmenge über einzelne Organe und Systeme des Körpers ergeben. Die akustischen Schwingungen bewirken jedoch die Entstehung einer Potentialdifferenz an den Grenzflächen zwischen einzelnen Medien mit unterschiedlichen akustischen Eigenschaften (Debye-Potential), die mit dem Membranpotential einer Vielzahl von Zellen vergleichbar ist, so dass dieses Verfahren nicht als völlig nicht-invasiv bezeichnet werden kann, und es treten Störungen zufolge von thermoelastischen Veränderungen von Geweben, insbesondere im Gehirn, auf, die die Messungen verzerren.

Weiters ist aus der RU 2 180 603C ein therapeutisches Gerät bekannt, bei dem rotierende Magnetfelder verwendet werden, um den menschlichen Körper zu beeinflussen. Zusätzlich dazu werden über Elektroden elektrische Spannungen an die Haut angelegt. Auf diese Weise kann eine Beeinflussung des Körpers in vielfältiger Weise erzielt werden. Es hat sich jedoch herausgestellt, dass die Gesamtwirkung über einen beschränkten Bereich nicht hinausgeht. Weitere Magnetvorrichtungen zur Behandlung des Körpers sind in der WO 99/39769 und in der RU 2 121 383C beschrieben. Auch hier treten ähnliche Nachteile auf.

Aufgabe der vorliegenden Erfindung ist es unter anderem, diese Nachteile zu vermeiden und eine Vorrichtung zu schaffen, die sowohl für diagnostische Zwecke als auch für therapeutische Zwecke einsetzbar ist und die auf nicht-invasivem Weg eine starke und reproduzierbare Wirkung auf den Körper auszuüben imstande ist.

Diese Aufgaben werden dadurch gelöst, dass im Wesentlichen koaxial zur Drehachse mindestens ein weiterer Magnet angeordnet ist, der gegensinnig zu den ersten Magneten orientiert ist. Eine derartige Vorrichtung ist aus der EP 0 838 236 A bekannt.

Bei der Anwendung der erfindungsgemäßen Vorrichtung im diagnostischen Bereich ist auf ein konzeptuell und in technischem Sinn ähnliches Verfahren zu verweisen, das als Voll-Verfahren bekannt ist und in Leonhardt H.: "Fundamentals in Electro Puncture according to Voll." ML-Verlag GesmbH, Hetzen, 1980; veröffentlicht worden ist. Die Idee dieses Verfahrens und dieser Vorrichtung liegt darin, einen ständig zu überwachenden Parameter zu definieren, der eine Unterscheidung zwischen den Reaktionen einer gesunden und einer kranken Person ermöglicht. In technischer Hinsicht besteht das Voll-Verfahren in der Definition der elektrischen Leitfähigkeit in verschiedenen Segmenten eines Körpermeridians und der Untersuchung der Dynamik in vorbestimmten Punkten des Körpers. Dabei wird der Messbereich auf eine Skala zwischen 0% und 100% abgebildet, wobei der Mittelpunkt der Skala einem Normwert entspricht, das obere Ende der Skala Entzündungskrankheiten zugeordnet wird, und das untere Ende der Skala einem Gewebeverfall zugeordnet wird. Die Anwendung des Voll-Verfahrens wird jedoch durch eine Reihe von technischen und organisatorischen Schwierigkeiten erschwert. Eine große Fehlerquelle stellt dabei die korrekte Anbringung von Elektroden an den vorbestimmten Körperpunkten dar, da geringfügige örtliche Veränderungen, die Geometrie der Elektrode, der aufgewendete Druck und andere Parameter relative große Einflüsse auf das Ergebnis haben. Darüber hinaus ist aus kybernetischer Hinsicht festzustellen, dass die vorbestimmten zu untersuchenden Körperpunkte zeitlich veränderliche, nicht-lineare, dynamische Objekte darstellen (Croley T.E.: "Electrical Acupuncture Point Conductance in the Compared to that in the Dead. Amer. J. Acupunct., 1986; 14(1):57-60), so dass die Ergebnisse der Diagnose nur den augenblicklichen Energiezustand des Körpers unter dem Einfluss von externen Faktoren wiederspiegeln können ohne zuverlässige Aussagen über den tatsächlichen grundlegenden Zustand des Organismus zuzulassen.

Die Vorrichtung der vorliegenden Erfindung ermöglicht es, den Körper in genau definierter Weise einem elektromagnetischen Feld auszusetzen, das eine robuste und zuverlässige Gewinnung von Messwerten ermöglicht, um den Körperzustand zu diagnostizieren. Durch den Ort der Anbringung und die Variation der Drehgeschwindigkeit bzw. der Frequenz von Drehrichtungsänderungen werden die Messwerte, die in an sich bekannter Weise über Elektroden, die am Körper angebracht sind, gewonnen werden, beeinflusst und verändert, und aus der Art der Beeinflussung können Rückschlüsse gezogen werden, die eine Gewinnung von zuverlässigen Diagnosen ermöglicht.

Ein wesentlicher Aspekt der Erfindung ist die gegensinnige Polarisierung des zentralen weiteren Magnets im Verhältnis zu den rotierenden ersten Magneten. Dies bedeutet, dass entweder der magnetische Nordpol des ersten Magneten zur ersten Gehäusewand weist und die magnetischen Südpole der ersten Magneten zur Gehäusewand weisen oder umgekehrt. Durch diese spezielle Anordnung kann das Magnetfeld, das in den Körper eindringt, in besonders zielgerichteter Weise auf einzelne Organe ausgerichtet werden. Die erste Gehäusewand ist aus einem magnetisch neutralen Material, beispielsweise Kunststoff, und möglichst dünn ausgeführt, um die Verluste so weit als möglich zu verringern. Bevorzugt werden als Magnete starke Magnete verwendet, die vorzugsweise eine Feldstärke zwischen 0,5 T und 5 T entwickeln.

Ein besonders einfacher Aufbau der erfindungsgemäßen Vorrichtung ergibt sich, wenn die Magnete als Permanentmagnete ausgebildet sind. Um die oben beschriebene Feldstärke sicher und zuverlässig erreichen zu können, ist es hierbei besonders bevorzugt, wenn die Magnete aus einer Neodym-Eisen-Bor-Legierung oder einer Praseodym-Eisen-Bor-Legierung hergestellt sind. Solche sogenannten SE-Magnete auf der Basis von seltenen Erden besitzen besonders vorteilhafte Eigenschaften in Zusammenhang mit der vorliegenden Erfindung. Alternativ dazu ist es auch möglich, die Magnete teilweise oder vollständig als Elektromagnete auszubilden. Die Versorgung solcher Elektromagnete auf dem Rotor kann in diesem Fall beispielsweise über Schleifringe erfolgen.

Durch geeignete Wahl der Drehzahl des Rotors (Winkelgeschwindigkeit) und gegebenenfalls periodische Veränderungen der Drehrichtung mit einer gewissen Basisfrequenz kann eine gezielte Beeinflussung des menschlichen aber auch des tierischen Körpers erfolgen. Dadurch können nicht nur im Zuge gleichzeitig durchgeführter Messungen präzise diagnostische Aussagen gewonnen werden, sondern auch therapeutische Behandlungen durchgeführt werden. Bei einer solchen Behandlung können beispielsweise Erkrankungen, die sich auf einer siebenstufigen Skala des energieinformativen Systems des Patienten darstellen und klassifizieren lassen, behandelt werden. Bei einer solchen Einteilung stellt die siebente Stufe den Zustand der Gesundheit dar, während niedrigere Stufen unterschiedliche Stadien von Erkrankungen definieren. Üblicherweise wird dabei zwischen der zweiten und der dritten Stufe ein Grenzwert angenommen, unterhalb dessen eine Rekonvaleszenz als unmöglich erscheint. Durch die Anwendung der erfindungsgemäßen Vorrichtung ist es möglich, einen Patienten von der dritten, vierten, fünften oder sechsten Stufe auf die siebente Stufe zu bringen. Doppelblindstudien, die an einer Vielzahl von Patienten durchgeführt worden sind, haben die Wirksamkeit der erfindungsgemäßen Vorrichtung erwiesen.

Erfindungsgemäß ist der weitere Magnet stationär am Gehäuse angebracht. Dies ermöglicht einen mechanisch besonders einfachen Aufbau der Vorrichtung, wobei der weitere Magnet beispielsweise direkt an der ersten Gehäusewand angebracht sein kann.

Als besonders günstig hat es sich herausgestellt, wenn die ersten Magnete im Bereich radialer Strahlen des Rotors befestigt sind, die gleichmäßige Winkelabstände aufweisen. Auf diese Weise kann ein mit gleichmäßiger Winkelgeschwindigkeit umlaufendes Magnetfeld erzeugt werden, wobei die Winkelgeschwindigkeit je nach Einsatzzweck entsprechend angepasst werden kann. Besonders bevorzugt ist die Anordnung der ersten Magnete auf drei Strahlen, so dass die Winkelabstände jeweils 120° betragen. Im einfachsten Fall kann entlang jedes Strahls ein Magnet angeordnet sein, bei größerflächiger Anwendung ist es jedoch bevorzugt, wenn auf jedem Strahl mehrere Magnete angeordnet sind.

Als besonders günstig hat es sich herausgestellt, wenn die ersten Magnete und der weitere Magnet Polflächen aufweisen, die in einer gemeinsamen Ebene liegen und die unmittelbar an die erste Gehäusewand angrenzen. Auf diese Weise wird eine besonders gleichmäßige Einwirkung des Magnetfelds auf das Gewebe erreicht.

In der Folge wird die vorliegende Erfindung anhand der in den Figuren dargestellten Ausführungsbeispiele näher erläutert. Es zeigen:
- Fig. 1: eine Ansicht des Rotors einer erfindungsgemäßen Vorrichtung,

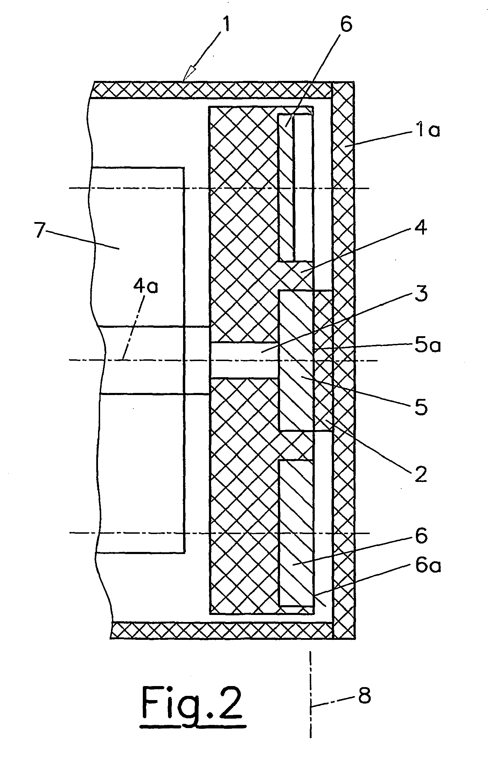

- Fig. 2: einen teilweisen Schnitt durch eine erfindungsgemäße Vorrichtung und
- Fig. 3: eine Ansicht entsprechend Fig. 1 einer alternativen Ausführungsvariante der Erfindung.

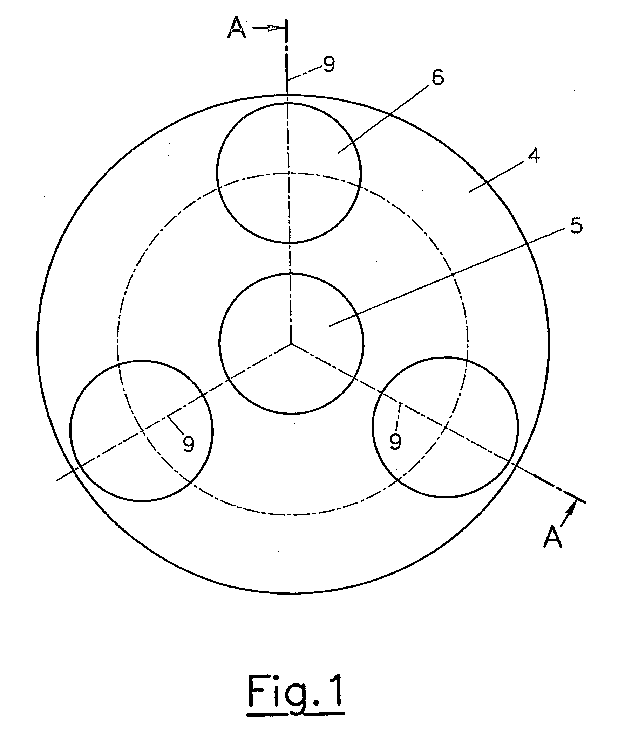

Die Vorrichtung von Fig. 1 besteht aus einem Gehäuse 1 mit einer im Wesentlichen ebenen Gehäusewand 1a aus Kunststoff. In dem Gehäuse 1 ist ein um eine Achse 4a drehbarer Rotor 4 angeordnet, der über eine Welle von einem Motor 7 angetrieben wird. An dem Rotor 4 sind insgesamt drei kreisrund ausgeführte erste Magnete 6 in gleichmäßigen Winkelabständen von 120° angeordnet. Koaxial zum Rotor 4 ist ein stationärer weiterer Magnet 5 vorgesehen, der über eine Zwischenplatte 2 fest mit der ersten Gehäusewand 1a verbunden ist. Die vorderen Polflächen 6a der ersten Magnete 6 und die vordere Polfläche 5a des weiteren Magnets 5 liegen in einer gemeinsamen Ebene 8, die in unmittelbarer Nachbarschaft der ersten Gehäusewand 1a angeordnet ist. Dabei entsprechen die vorderen Polflächen 6a der ersten Magnete 6 jeweils dem magnetischen Nordpol und die vordere Polfläche 5a des weiteren Magnets 5 dem magnetischen Südpol. Nicht dargestellt sind entsprechende Steuerungseinrichtungen, die einen Antrieb des Rotors 4 mit unterschiedlichen Winkelgeschwindigkeiten und in wechselnder Drehrichtung ermöglichen.

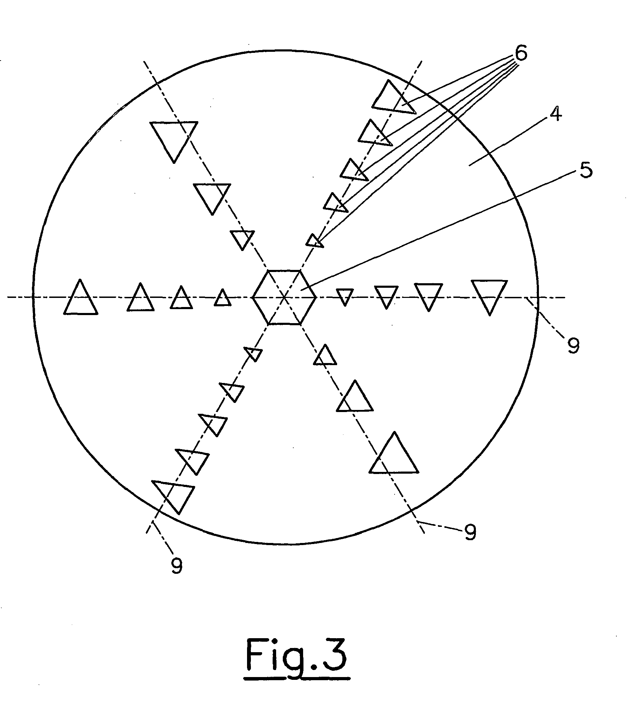

Eine alternative Ausführungsvariante ist in Fig. 3 dargestellt, bei der die ersten Magnete auf insgesamt sechs Strahlen 9 in gleichmäßigen Winkelabständen von 60° angeordnet sind, und wobei auf jeden Strahl 9 mehrere, im vorliegenden Fall fünf, erste Magnete 6 angeordnet sind.

## Patentansprüche

1. Vorrichtung zur Erfassung bzw. Beeinflussung des physiologischen und ggf. pathologischen Zustands des menschlichen oder tierischen Körpers mit einem Gehäuse (1), das eine erste Gehäusewand (1a) aufweist, die eine Außenfläche besitzt, die dazu bestimmt ist, an dem zu behandelnden Körper angelegt zu werden, wobei in dem Gehäuse (1) ein Rotor (4) angeordnet ist, der um eine Achse (4a) drehbar angetrieben ist, die im Wesentlichen senkrecht zu der ersten Gehäusewand (1a) ist, und wobei auf dem Rotor (4) erste Magnete (6) angeordnet sind, deren Magnetfelder gleichsinnig parallel zur Drehachse orientiert sind wobei weiters im Wesentlichen koaxial zur Drehachse (4a) mindestens ein weiterer Magnet (5) angeordnet ist, der gegensinnig zu den ersten Magneten (6) orientiert ist, **dadurch gekennzeichnet, dass** der weitere Magnet stationär am Gehäuse (1) angebracht ist.

2. Vorrichtung nach Anspruch 1, **dadurch gekennzeichnet, dass** die ersten Magnete im Bereich radialer Strahlen (9) des Rotors (4) befestigt sind, die gleichmäßige Winkelabstände aufweisen.

3. Vorrichtung nach Anspruch 2, **dadurch gekennzeichnet, dass** die Winkelabstände jeweils 120° betragen.

4. Vorrichtung nach einem der Ansprüche 2 oder 3, **dadurch gekennzeichnet, dass** entlang jedes Strahls (9) mehrere erste Magnete (6) angeordnet sind.

5. Vorrichtung nach einem der Ansprüche 2 oder 3, **dadurch gekennzeichnet, dass** entlang jedes Strahls (9) genau ein erster Magnet (6) angeordnet ist.

6. Vorrichtung nach einem der Ansprüche 1 bis 5, **dadurch gekennzeichnet, dass** die ersten Magnete (6) und der weitere Magnet (5) Polflächen (5a; 6a) aufweisen, die in einer gemeinsamen Ebene (8) liegen und die unmittelbar an die erste Gehäusewand (1a) angrenzen.

7. Vorrichtung nach einem der Ansprüche 1 bis 6, **dadurch gekennzeichnet, dass** der Rotor (4) durch einen Antriebsmotor (7) angetrieben ist, der auf verschiedene Drehzahlen und Drehrichtungen einstellbar ist.

8. Vorrichtung nach einem der Ansprüche 1 bis 7, **durch gekennzeichnet, dass** die ersten Magnete (6) und der weitere Magnet (5) als Permanentmagnete ausgebildet sind.

9. Vorrichtung nach einem der Ansprüche 1 bis 7, **durch gekennzeichnet, dass** die ersten Magnete (6) und der weitere Magnet (5) als Elektromagnete ausgebildet sind.

## Claims

1. Device for detecting and influencing the physiological and, optionally, the pathological state of the human or animal body, comprising a housing (1), which has a first housing wall (1a) having an outer face, which is intended to be applied to the body to be treated, wherein a rotor (4) is arranged in the housing (1) and is rotatably driven about an axis (4a), which is substantially perpendicular to the first housing wall (1a), and wherein first magnets (6), the magnetic fields of which are oriented in the same direction parallel to the rotation axis, are arranged on the rotor (4), wherein, furthermore, at least one further magnet (5) is arranged substantially coaxially to the rotation axis (4a) and is oriented in the opposite direction to the first magnets (6), **characterised in that** the further magnet is provided stationary on the housing (1).

2. Device according to claim 1, **characterised in that** the first magnets are fastened in the region of radial rays (9) of the rotor (4), which have uniform angular spacings.

3. Device according to claim 2, **characterised in that** the angular spacings are 120°, in each case.

4. Device according to either of claims 2 or 3, **characterised in that** a plurality of first magnets (6) are arranged along each ray (9).

5. Device according to either of claims 2 or 3, **characterised in that** precisely one first magnet (6) is arranged along each ray (9).

6. Device according to any one of claims 1 to 5, **characterised in that** the first magnets (6) and the further magnet (5) have pole faces (5a; 6a), which lie in a common plane (8) and directly adjoin the first housing wall (1a).

7. Device according to any one of claims 1 to 6, **characterised in that** the rotor (4) is driven by a drive motor (7), which can be adjusted to various rotational speeds and rotational directions.

8. Device according to any one of claims 1 to 7, **characterised in that** the first magnets (6) and the further magnet (5) are configured as permanent magnets.

9. Device according to any one of claims 1 to 7, **characterised in that** the first magnets (6) and the further magnet (5) are configured as electromagnets.

## Revendications

1. Dispositif pour la saisie, respectivement l'influencement, de l'état physiologique et, le cas échéant, pathologique du corps humain ou animal, avec un boîtier (1) qui comporte une première paroi de boîtier (1a), qui présente une face extérieure, laquelle est destinée à être appliquée sur le corps à traiter, un rotor (4) étant disposé dans le boîtier (1), lequel rotor étant entraîné an rotation autour d'un axe (4a), qui est essentiellement perpendiculaire à la première paroi de boîtier (1a), et dans lequel, sur le rotor (4) sont disposés des premiers aimants (6), dont les champs magnétiques sont orientés dans le même sens parallèlement à l'axe de rotation et, en outre, au moins un autre aimant (5) est disposé essentiellement coaxialement par rapport à l'axe de rotation (4a), qui est orienté en sens contraire des premiers aimants (6), **caractérisé en ce que** l'autre aimant est monté de façon stationnaire sur le boîtier (1).

2. Dispositif selon la revendication 1, **caractérisé en ce que** les premiers aimants sont fixés dans la zone de rayons radiaux (9) du rotor (4) qui présentent des écartements angulaires réguliers.

3. Dispositif selon la revendication 2, **caractérisé en ce que** les écartements angulaires sont chacun de 120°.

4. Dispositif selon l'une des revendications 2 ou 3, **caractérisé en ce que** plusieurs premiers aimants (6) sont disposés le long de chaque rayon (9).

5. Dispositif selon l'une des revendications 2 ou 3, **caractérisé en ce qu'**un premier aimant (6) exactement est disposé le long de chaque rayon (9).

6. Dispositif selon l'une des revendications 1 à 5, **caractérisé en ce que** les premiers aimants (6) et l'autre aimant (5) comportent des surfaces polaires (5a; 6a) qui se trouvent dans un plan (8) commun et qui sont directement contiguës à la première paroi de boîtier (1a).

7. Dispositif selon l'une des revendications 1 à 6, **caractérisé en ce que** le rotor (4) est entraîné par un moteur d'entraînement (7) qui peut être réglé à différentes vitesses de rotation et à différents sens de rotation.

8. Dispositif selon l'une des revendications 1 à 7, **caractérisé en ce que** les premiers aimants (6) et l'autre aimant (5) sont configurés comme aimants permanents.

9. Dispositif selon l'une des revendications 1 à 7, **caractérisé en ce que** les premiers aimants (6) et l'autre aimant (5) sont configurés comme électroaimants.
